# FASCICULE DE BREVET EUROPEEN

(11) **EP 0 435 983 B1**
(45) Date de publication et mention de la délivrance du brevet: **03.11.1993**
(21) Numéro de dépôt: 90910091.9
(22) Date de dépôt: 16.07.1990
(51) Int. Cl.: A61F 2/02

(54) **PROTHESE POUR EMPECHER LES REFLUX GASTRIQUES DANS L'OESOPHAGE**
PROTHESE ZUM VERHINDERN EINES GASTRISCHEN RÜCKFLUSSES IN DIE SPEISERÖHRE
PROSTHESIS FOR PREVENTING THE GASTRIC REFLUX IN THE OESOPHAGUS

(30) Priorité: 20.07.1989 CH 2703/89
(43) Date de publication de la demande: 10.07.1991
(73) Titulaire: GODIN, Norman, 1254 Jussy (CH)
(72) Inventeur: GODIN, Norman, 1254 Jussy (CH)
(74) Mandataire: Dousse, Blasco (CH)
(86) Numéro de dépôt international: CH9000171
(87) Numéro de publication internationale: WO9101117

(56) Documents cités:
- US-A- 4 265 694
- US-A- 4 434 810
- US-A- 4 846 836

## Description

La présente invention se rapporte à une prothèse pour empêcher les reflux gastriques dans l'oesophage, comprenant une valve associée à une partie annulaire de fixation et présentant une ouverture dont la section de passage est contrôlée par des moyens élastiques. Une telle prothèse est connue du document US-A-4 846 836.

L'oesophagite est provoquée par des reflux gastriques chroniques. Si la muqueuse de l'estomac est apte à supporter le pH très acide des secrétions gastriques qui est voisin de 1, tel n'est pas le cas de celle de l'oeophage. Par conséquent, lorsque ces reflux sont chroniques, ils attaquent la muqueuse de l'oesophage et créent des ulcères qui, à la longue, peuvent entraîner un rétrécissement du conduit oesophagien.

Ces reflux gastriques sont associés, en général, à une hernie hiatale. La thérapeutique la plus couramment pratiquée dans ce genre d'affection a recours aux médicaments. Il en existe de trois catérogies, les antacides qui tendent à rendre le milieu neutre par apport d'un produit alcalin, les anti-histaminiques H₂ qui se fixent sur le récepteur H₂ de la cellule pariétale. Dernièrement, on a proposé un nouveau médicament qui, lui, bloque la production de ions H⁺ par la cellule pariétale. Toutefois, ce médicament n'a plus d'effet dès qu'on cesse de l'administrer et il ne peut pas être pris en continu dans la mesure où il peut provoquer des tumeurs, constatées tout au moins chez le rat. Enfin, la troisième classe se compose des médicaments qui augmentent la motricité de l'oesophage et de l'estomac et tendent à réduire le temps de contact des reflux acides avec l'oesophage. Cette thérapeutique ne s'attaquant pas à la cause première du mal, qui est le reflux gastrique, celui-ci réapparaît sitôt l'arrêt de la thérapeutique médicamenteuse, de sorte que le malade se voit contraint de prendre ces médicaments de façon permanente. Cette solution n'est évidemment satisfaisante, ni sur le plan médical, ni sur le plan économique.

Il a déjà été proposé, comme alternative à cette voie médicamenteuse, l'utilisation d'une prothèse externe destinée à s'opposer mécaniquement aux reflux gastriques. Cette prothèse externe est formée par un anneau élastiquement extensible, disposé autour de l'extrémité où l'oesophage débouche dans l'estomac. En enserrant ainsi la base de l'oesophage, la force centripète exercée par cet anneau oppose une résistance à l'écoulement qui tend à empêcher que les reflux gastriques ne remontent dans l'oesophage. Toutefois, l'effet de cet anneau se manifeste aussi bien vis-à-vis du reflux gastrique que vis-à-vis de la déglutition du bol alimentaire. Par conséquent, la pression centripète ne peut pas être choisie trop élevée sous peine de causer une gêne inaceptable à la déglutition. L'absence de sélectivité de cette solution vis-à-vis du sens de l'écoulement ne permet pas de garantir une efficacité totale de cette prothèse externe. Il a en outre été constaté que la prothèse externe peut être poussée vers le haut sous la pression du reflux gastrique, de sorte que la base de l'oesophage se trouve de nouveau exposée à l'attaque par l'acidité des liquides gastriques. Cet anneau se trouvant à l'extérieur de l'oesophage, sa position ne peut plus être modifiée par endoscopie. Les déplacements de cette prothèse externe dans la cavité abdominale limitent son emploi et peuvent présenter des dangers.

Ces inconvénients expliquent pourquoi l'utilisation de cette prothèse ne s'est pas généralisée, dans la mesure où elle n'offre pas de garantie suffisante. En cas d'échec, il faut de nouveau recourir aux médicaments et la proportion d'échecs s'est révélée importante.

Enfin il existe également les procédés chirurgicaux, notamment la fundoplicature selon Nissen-Rossetti qui consiste à fabriquer un manchon avec le fundus gastrique autour du cardia, sous le diaphragme. L'inconvénient d'une telle intervention est que, en cas de péristaltisme oesophagien déficient, elle peut provoquer une dysphagie sévère. Il existe encore d'autres procédés chirurgicaux. Toutefois, toutes les solutions chirurgicales présentent des risques post-opératoires tel que le reflux récidivant suite au lâchage des sutures, la dysphagie lorsque le manchon est trop étroit, le glissement d'une partie de l'estomac en amont du manchon provoquant alors une sévère oesophagite de reflux. En outre, le patient ainsi opéré ne peut alors ni éructer ni vomir, ce que certains supportent difficilement.

On a également déjà proposé, dans le US-A-4,846,836, une prothèse en un matériau élastomère, destinée à être placée dans l'oesophage et comprenant un cône à l'intérieur d'une partie tubulaire destinée à la fixation de la prothèse. Le cône a son sommet fendu et dirigé vers l'estomac, constituant une sorte d'entonnoir qui se termine par une valve dont la fente est destinée à s'ouvrir sous la poussée péristaltique exercée sur le bol alimentaire, mais à empêcher l'écoulement en sens inverse. Une seconde fente, ménagée entre la base du cône et la partie tubulaire, est destinée à s'ouvrir sous une certaine pression de reflux pour permettre le vomissement.

Un inconvénient majeur de cette prothèse provient du fait que le bol alimentaire doit passer à travers une section sensiblement réduite de la valve ménagée au sommet du cône, ce cône étant nécessaire pour permettre à l'écoulement inverse d'accéder à la seconde ouverture destinée au reflux en cas de vomissement. Il est évident qu'une telle valve constitue une gêne certaine pour le patient qui aura du mal à déglutir, en particulier les solides, en raison du rétrécissement au passage de la fente, ce qui peut provoquer des douleurs difficilement supportables.

Le but de la présente invention est de remédier, au moins en partie, aux inconvénients des solutions susmentionnées.

A cet effet, cette invention a pour objet une prothèse pour empêcher les reflux gastriques dans l'oesophage selon la revendication 1.

L'avantage essentiel de la solution proposée provient du fait que la même valve, tout en ayant un effet unidirectionnel, permet le reflux lorsque la pression est suffisante, c'est-à-dire en cas de vomissement. Cette valve a aussi l'énorme avantage d'offrir, en position ouverte, une section de passage sensiblement égale à celle de l'oesophage, permettant une déglutition aisée des aliments.

Le dessin annexé illustre, schématiquement et à titre d'exemple,une forme d'exécution et deux variantes de la prothèse objet de la présente invention.

La figure 1 est une vue en perspective avec coupe d'une forme d'exécution de cette prothèse fixée à la base de l'oeosophage.

La figure 2 est une vue en élévation de la forme d'exécution de la figure 1.

La figure 3 est une vue selon la ligne III-III de la figure 2.

La figure 4 est une vue en élévation d'une variante des figures 1 à 3.

La figure 5 est une vue en coupe selon la ligne V-V de la figure 4.

La figure 6 est une vue selon la ligne VI-VI de la figure 5.

La figure 7 est une vue semblable à la figure 4, d'une variante.

La figure 8 est une vue en coupe selon la ligne VIII-VIII de la figure 7.

La figure 9 est une vue selon IX-IX de la figure 7.

La figure 10 est une vue en perspective de la variante des figures 7-9 disposée dans une hernie hiatale.

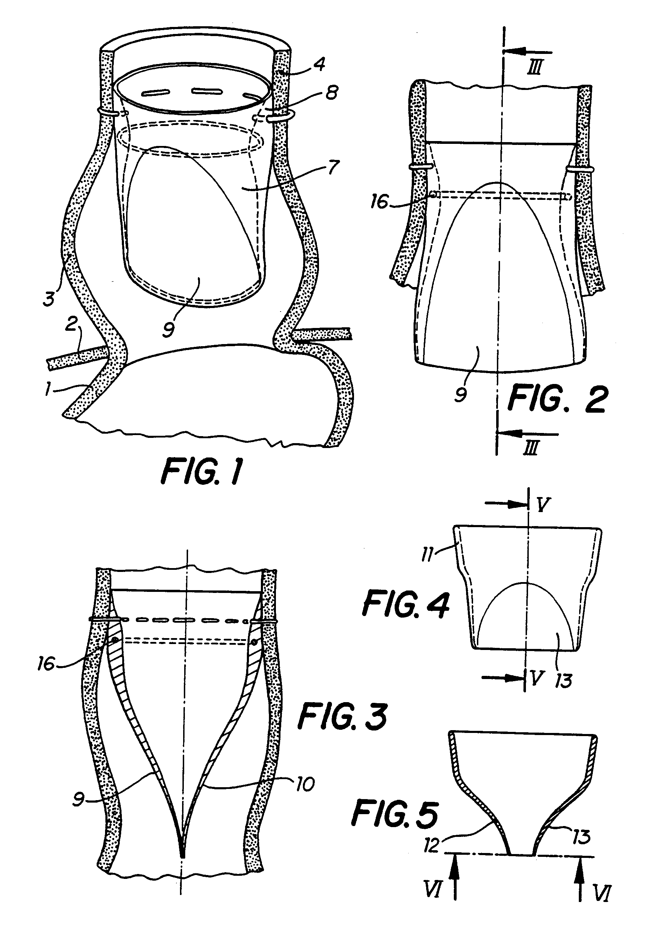

La figure 1 montre le haut de l'estomac 1 qui présente, au-dessus du diaphragme 2, une hernie hiatale 3. Bien que le reflux gastrique ne soit pas toujours lié à la présence d'une telle hernie, il s'agit tout de même de la cause la plus fréquente de cette affection. La base de l'oesophage 4 débouche dans cette hernie hiatale 3.

La forme d'exécution illustrée par les figures 1-3 montre une valve formée d'une partie tubulaire 7 associée à un élément annulaire de fixation 8. Cette partie tubulaire qui peut d'ailleurs elle-même former l'élément de fixation, s'aplatit ensuite progressivement pour former deux lèvres jointives 9 et 10. Cette solution offre une très faible résistance au passage du bol alimentaire et nécessite peu ou pas de capacité d'extension, dans la mesure où il suffit que les lèvres 9 et 10 s'écartent l'une de l'autre pour livrer passage à l'écoulement. On peut réaliser cette prothèse en un élastomère à base de silicone à deux composants de qualité médicale, vendu sous la marque Silastsic® par Dow Corning Corp., ou en un polymere biocompatible tel que celui décrit dans le US-A-4,657,544 ou dans le US-A-4,759,757 pourrait convenir pour une telle application. Il s'agit d'un polymère greffé sans solvant à deux composants hydrophiles ou hydrophobes, dans lequel on incorpore un sel inorganique soluble dans l'eau, qui a été broyé et tamisé. Un tube est alors formé et les cristaux du sel sont lessivés du tube ainsi formé pour ménager une structure en nid d'abeilles qui augmente la flexibilité et permet d'améliorer les propriétés de collage au cas où ce mode de fixation est utilisé. Outre les élastomère susmentionnés, on peut encore citer les composés fluoro-élastomères (par exemple Viton® ) ainsi que les caoutchoucs de type butyle. Il est possible de former la valve avec une épaisseur de paroi qui augmente de l'extrémité libre des lèvres 9 et 10 en direction de la partie annulaire de fixation 8 pour éviter le retournement trop facile sous l'effet de la pression de reflux. On peut remarquer que cette forme d'exécution offre une grande surface sur laquelle la pression de reflux peut agir pour fermer les lèvres 9 et 10. Si la partie tubulaire est un peu plus rigide en raison de son épaisseur accrue, la valve travaille essentiellement par écartement et resserrement des lèvres 9 et 10.

Comme on peut encore l'observer sur les figures 1-3, un fil métallique très fin 16 peut être noyé dans l'élément annulaire de fixation en vue de permettre le repérage radiologique de la position de la valve.

D'autres formes d'exécution sont envisageables basées sur ce même concept. La variante illustrée par les figures 4 et 5 se distingue essentiellement par le fait que l'aplatissement de la partie annulaire 11 pour former les lèvres 12 et 13 est beaucoup plus brusque, réduisant ainsi la dimension axiale de la valve. Cette réduction de dimension présente l'avantage de prendre moins de place et de permettre de loger la prothèse tout entière dans pratiquement toutes les hernies hiatales. Dans cet exemple, la dimension axiale de la prothèse est de l'ordre de 15 à 20 mm. Mais la différence principale de cette variante réside dans le fait qu'en position de repos, les deux lèvres 12 et 13 restent entrouvertes, comme illustré par la figure 5, de manière à faciliter le passage du bol alimentaire et à éviter que des restes de nourriture et la salive ne séjournent dans la valve. En maintenant les lèvres 12 et 13 entrouvertes, ce risque est pratiquement évité et la salive peut s'écouler dans l'estomac sans l'aide d'aucune force pour écarter les lèvres 12 et 13. Dans cette variante, l'épaisseur de la paroi au niveau de l'extrémité des lèvres 12 et 13 est de l'ordre de 0,2 à 0,4 mm, tandis qu'elle s'épaissit pour atteindre 1,2 à 1,7 mm dans la partie annulaire 11.

En cas de reflux d'acide gastrique, la pression qui s'exerce sur les faces externes des parties aplaties qui forment les lèvres 12 et 13 provoque la fermeture de ces lèvres. Même si une faible quantité d'acide gastrique passait entre ces lèvres, ce reflux ne risque pas de dépasser le niveau de la valve et donc de s'attaquer à la muqueuse de l'oesophage. Dès que la pression de reflux disparaît, les lèvres 12 et 13 s'écartent à nouveau dans la position de repos illustrée par la figure 5 et laissent redescendre l'acide qui pourrait se trouver dans la valve.

Si la pression de reflux vient à augmenter sensiblement, ce qui ne se produit qu'en cas de vomissement, les lèvre 12 et 13 se retournent et laissent passer l'écoulement en sens inverse. Dans la variante illustrée par les figures 4 et 5, on a constaté qu'en cas de retournement, ces lèvres reviennent dans leur position initiale par leur propre élasticité.

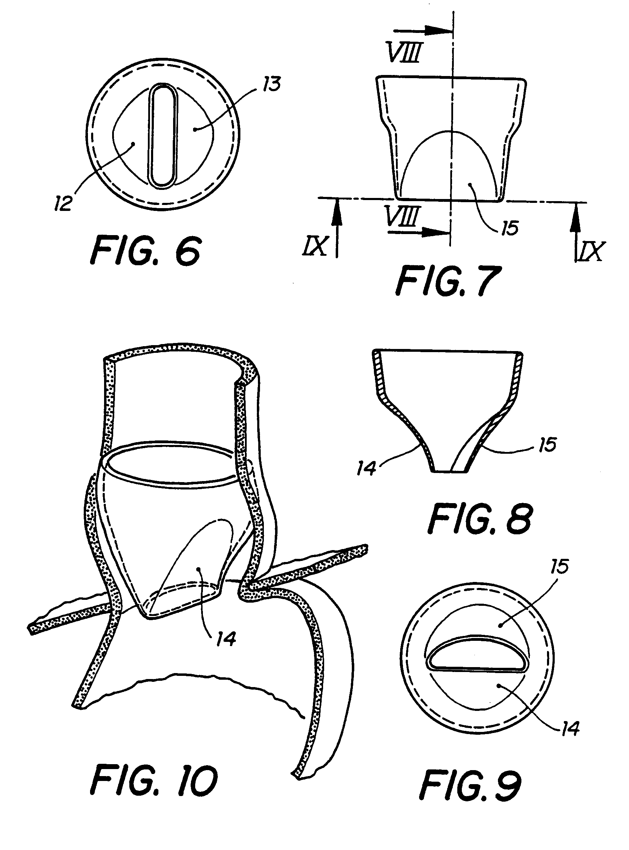

La seconde variante illustrée par les figures 7 à 10 diffère de celle des figures 4 et 5 par le fait que les lèvres 14 et 15 sont asymétriques, ménageant entre elles une ouverture en forme de D au lieu du O allongé formé par l'ouverture située entre les lèvres symétriques 12 et 13. La raison du choix de cette forme asymétrique de l'ouverture ménagée entre les lèvres 14 et 15 provient du fait que l'estomac a lui-même une forme asymétrique comme illustré par la figure 10, de sorte que la pression de reflux qui s'exerce sur les lèvres 14 et 15 n'est pas verticale, mais plutôt latérale et que la lèvre 14 est ainsi soumise à une pression plus élevée que la lèvre 15. En donnant une forme convexe à la lèvre 15, celle-ci facilite encore le passage du bol alimentaire. Par contre la lèvre droite 14 offre une plus faible résistance à la déformation et comme elle est soumise à la plus grande pression de reflux qui vient du côté droit, elle est appliquée contre la lèvre 15 et ferme l'orifice de passage en cas de reflux, la lèvre 15 restant pratiquement immobile.

## Revendications

1. Prothèse pour empêcher le reflux gastrique dans l'oesophage, comprenant une valve associée à une partie annulaire de fixation (8) et présentant une ouverture dont la section de passage est contrôlée par des moyens élastiques, caractérisée par le fait que cette valve est ménagée à partir d'un élément de forme générale tubulaire (7) en un matériau élastiquement déformable et dont la section est progressivement déformée de façon permanente pour resserrer la paroi de cet élément à l'une de ses extrémités formant ainsi une valve en bec de canard, de sorte qu'en position d'écartement maximum de la paroi à cette extrémité, une ouverture correspondant sensiblement à la section de la partie tubulaire située a l'autre extrémité dudit élément soit ménagée, cette extrémité resserrée étant destinée à être placée en aval de la partie tubulaire dudit élément pour que toute force qui est appliquée sur la face interne de la paroi de ladite extrémité resserrée dudit élément tubulaire et qui est engendrée par l'onde péristaltique de l'oesophage exercée sur le bol alimentaire pendant la déglutition tende à écarter ces parois, tandis que toute force de sens contraire qui s'exerce en cas de reflux sur les faces externes de la paroi de ladite extrémité resserrée de l'élément tubulaire tende à les rendre jointives tant qu'elle ne dépasse pas une limite sensiblement supérieure à celle engendrée par l'onde péristaltique de l'oesophage provoquant ainsi le retournement au moins partiel de l'élément tubulaire.

2. Prothèse selon la revendication 1, caractérisée par le fait que l'extrémité resserrée de la paroi dudit élément ménage une ouverture de section réduite en position de repos.

3. Prothèse selon la revendication 1, caractérisée par le fait que la paroi dudit élément s'amincit progressivement en direction de ladite extrémité resserrée.

## Claims

1. A prosthesis to prevent gastric reflux in the oesophagus, comprising a valve associated with an annular fixing portion (8) and having an opening whose opening area is controlled by resilient means, characterised by the fact that this valve is made from an element (7) of generally tubular shape which is made of a resiliently deformable material and the cross-section of which is deformed progressively in a permanent manner so as to constrict the wall of this element at one of its ends thus forming a duck-bill valve such that in the position of maximum spacing apart of the wall at this end there is produced an opening substantially corresponding to the cross-section of the tubular portion located at the other end of the said element, this constricted end being intended to be placed downstream of the tubular portion of the said element in order that all force which is applied to the internal face of the wall of the said constricted end of the said tubular element and is produced by the peristaltic wave of the oesophagus exerted on the alimentary bolus during deglutition tends to spread these walls apart, while all force in the opposite direction which is exerted on the external faces of the wall of the said constricted end of the tubular element in the event of reflux tends to render them contiguous as long as it does not exceed a limit substantially higher than that produced by the peristaltic wave of the oesophagus and thus causes the at least partial inversion of the tubular element.

2. A prosthesis in accordance with Claim 1, characterised by the fact that the constricted end of the wall of the said element forms an opening of reduced cross-section in the rest position.

3. A prosthesis in accordance with Claim 1, characterised by the fact that the wall of the said element becomes progressively thinner in the direction of the said constricted end.

## Patentansprüche

1. Prothese zum Verhindern des gastrischen Rückflusses in die Speiseröhre, welche Prothese ein Ventil besitzt, das einem ringförmigen Befestigungsteil (8) zugeordnet ist und eine Öffnung hat, deren Durchtrittsquerschnitt durch elastische Mittel gesteuert wird, dadurch gekennzeichnet, daß dieses Ventil aus einem Element mit der Grundform eines allgemeinen Rohres (7) aus elastisch deformierbaren Material gebildet ist, dessen Querschnitt zunehmend in bleibender Art deformiert ist, um die Wandung dieses Elementes an einem seiner Enden zu verengen und so ein entenschnabelähnliches Ventil derart zu bilden, daß an der Stelle des maximalen Abstandes der Wandung an diesem Ende eine Öffnung gebildet ist, die im wesentlichen dem Querschnitt des Rohrteiles am anderen Ende des Elementes entspricht, wobei dieses verengte Ende in Flußrichtung hinter dem rohrförmigen Abschnitt des Elementes angeordnet ist, damit jegliche Kraft, die auf die innere Fläche der Wandung dieses verengten Endes des rohrförmigen Elementes aufgebracht wird und die durch die peristaltische Welle der Speiseröhre erzeugt wird, welche Kraft auf die Nahrungsmittelkugel während des Schluckens ausgeübt wird, versucht, die Wände zu spreizen, wohingegen jegliche Kraft in entgegengesetzter Richtung, die im Falle eines Rückflusses auf die äußeren Flächen der Wand dieses verengten Endes des rohrförmigen Elementes ausgeübt wird, versucht sie zusammenzuhalten, damit sie nicht eine Grenze, die geringfügig größer ist als jene, die die peristaltische Welle der Speiseröhre erzeugt, überschreitet und so wenigstens teilweise die Rückführung des rohrförmigen Elementes hervorruft.

2. Prothese nach Anspruch 1, dadurch gekennzeichnet, daß das verengte Ende der Wand des Elementes eine Öffnung mit verringertem Querschnitt in Ruhelage bildet.

3. Prothese nach Anspruch 1, dadurch gekennzeichnet, daß die Wand des Elementes zunehmend in Richtung des verengten Endes dünner wird.
